# EUROPEAN PATENT APPLICATION

(11) **EP 4 378 924 A1**
(43) Date of publication of application: **05.06.2024**
(21) Application number: 22869863.5
(22) Date of filing: 06.09.2022
(51) Int. Cl.: C07C 231/02, C07C 233/09, C07C 233/20

(54) **PRODUCTION METHOD FOR (METH)ACRYLAMIDE COMPOUND**

(30) Priority: 17.09.2021 JP 2021152361
(71) Applicant: MITSUI CHEMICALS, INC., Tokyo 104-0028 (JP)
(72) Inventor: KAKINUMA, Naoyuki, Sodegaura-shi, Chiba 299-0265 (JP)
(74) Representative: Hoffmann Eitle
(86) International application number: PCT/JP2022/033484
(87) International publication number: WO 2023/042716

(57) **Abstract**

A method for producing a (meth)acrylamide compound, comprising synthesizing a (meth)acrylamide compound (X) by reacting a (meth)acrylic acid with a primary amine compound in the presence of a condensing agent.

## Description

### Technical Field

The present disclosure relates to a method for producing a (meth)acrylamide compound.

### Background Art

A (meth)acrylate compound is widely used as a monomer in a curable composition.

For example, Patent Literature 1 discloses a (meth)acrylate (D) as a monomer which can provide a cured product having both high toughness and rigidity. The (meth)acrylate (D) in this literature is a reaction product of a thiol compound (A) having two or more mercapto groups, an iso(thio)cyanate compound (B) having two or more iso(thio)cyanate groups, and a hydroxy (meth)acrylate compound (C) having one or more polymerizable groups. This literature also discloses a composition containing the (meth)acrylate (D).

Patent Literature 1: WO 2019/107323 A1

### SUMMARY OF INVENTION

### Technical Problem

The (meth)acrylate compound is known as a monomer exhibiting polymerizability. For example, the (meth)acrylate compound may be used as a monomer contained in a monomer composition for a dental material.

The (meth)acrylate compound is a monomer used for various applications, and is required to be produced by a simple production method.

An object of one aspect of the disclosure is to provide a simple method for producing a (meth)acrylamide compound.

### Solution to Problem

Means for solving the above problems include the following aspects.
<1> A method for producing a (meth)acrylamide compound, including synthesizing a (meth)acrylamide compound (X) by reacting a (meth)acrylic acid with a primary amine compound in the presence of a condensing agent.
<2> The method for producing a (meth)acrylamide compound according to <1>, in which the condensing agent is a carbodiimide compound represented by the following Formula (Z) or a hydrochloride of a carbodiimide compound containing an amino group among carbodiimide compounds represented by the following Formula (Z). 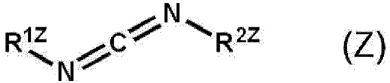 In Formula (Z), each of R^{1Z} and R^{2Z} independently represents a hydrocarbon group having from 1 to 10 carbon atoms that may be substituted with an amino group.
<3> The method for producing a (meth)acrylamide compound according to <1> or <2>, in which the condensing agent is a carbodiimide compound represented by the following Formula (Z1) or a hydrochloride thereof. 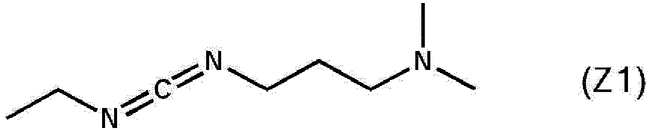
<4> The method for producing a (meth)acrylamide compound according to any one of <1> to <3>, in which the primary amine compound is a diamine compound.
<5> The method for producing a (meth)acrylamide compound according to any one of <1> to <4>, in which the synthesizing a (meth)acrylamide compound (X) obtains a composition containing the (meth)acrylamide compound (X), and the method further includes, after the synthesizing a (meth)acrylamide compound (X), subjecting the composition containing the (meth)acrylamide compound (X) to acid washing and alkali washing.

### Advantageous Effects of Invention

According to one aspect of the disclosure, it is possible to provide a simple method for producing a (meth)acrylamide compound.

### DESCRIPTION OF EMBODIMENTS

In the disclosure, a numerical range that has been indicated by use of "to" indicates the range that includes the numerical values which are described before and after "to", as a lower limit value and an upper limit value, respectively.

In the disclosure, the term "step" includes not only an independent step but also a step by which an intended action of the step is achieved, though the step cannot be clearly distinguished from other steps.

In a case in which a plurality of substances corresponding to each component are present in a composition, the content of each component in the composition in the disclosure means the total content of the plurality of substances present in the composition unless otherwise specified.

In a numerical range described in a stepwise manner in the disclosure, an upper limit value or a lower limit value described in one numerical range may be replaced with an upper limit value or a lower limit value described in another numerical range described in a stepwise manner. In a numerical range described in the disclosure, an upper limit value or a lower limit value of the numerical range may be replaced with a value shown in Examples.

In the disclosure, "light" is a concept including active energy rays such as ultraviolet rays or visible rays.

In the disclosure, "(meth)acrylic compound" means an acrylic compound or a methacrylic compound, "(meth)acrylate" means acrylate or methacrylate, and "(meth)acrylic acid" means acrylic acid or methacrylic acid.

### «Method for Producing (Meth)Acrylamide Compound»

A method for producing a (meth)acrylamide compound of the disclosure (also simply referred to as the production method of the disclosure) includes synthesizing a (meth)acrylamide compound (X) by reacting a (meth)acrylic acid with a primary amine compound in the presence of a condensing agent (also referred to as a synthesis step).

Conventionally, as the method for producing a (meth)acrylamide compound, for example, an acid chloride method of reacting (meth)acrylic acid chloride with a primary amine compound has been often used. In the case of the acid chloride method, there is a problem in that it takes time for purification to obtain a highly pure (meth)acrylamide compound, or it is necessary to use a column for purification, which takes time and effort.

On the other hand, the production method of the disclosure is a method in which a (meth)acrylic acid and a primary amine compound are subjected to a condensation reaction in the presence of a condensing agent. As the condensing agent, for example, carbodiimide is suitably used.

By using the production method of the disclosure, a (meth)acrylamide compound can be easily produced.

The production method of the disclosure can also suppress coloring of a composition containing the (meth)acrylamide compound (X) obtained by reacting a (meth)acrylic acid with a primary amine compound.

The production method of the disclosure does not necessarily require a complicated purification treatment (for example, column purification) to be performed on the composition containing the (meth)acrylamide compound (X) to be obtained. In the production method of the disclosure, for example, purification can also be performed by water washing.

This point is one of factors for improving convenience in the production method of the disclosure.

Since the production method of the disclosure does not require a complicated purification treatment, the yield of the (meth)acrylamide compound as a product is also excellent.

### <Synthesis Step>

The synthesis step is to synthesize a (meth)acrylamide compound (X) by reacting a (meth)acrylic acid with a primary amine compound in the presence of a condensing agent.

A composition containing the (meth)acrylamide compound (X) may be obtained by the synthesis step.

The reaction time for reacting the (meth)acrylic acid with the primary amine compound is preferably from 3 to 20 hours, more preferably from 5 to 15 hours, and still more preferably from 6 to 10 hours.

The reaction temperature at which the (meth)acrylic acid is reacted with the primary amine compound is preferably from 0°C to 50°C, more preferably from 5°C to 40°C, and still more preferably from 10°C to 40°C.

### (Condensing Agent)

The production method of the disclosure uses a condensing agent.

The condensing agent in the disclosure is not particularly limited, and examples thereof include a carbodiimide-based condensing agent such as N,N'-dicyclohexylcarbodiimide, an imidazole-based condensing agent such as N,N'-carbonyldiimidazole, a triazine-based condensing agent such as 4-(4,6-dimethoxy-1,3,5-triazine-2-yl)-4-methylmorpholinium=chloride n-hydrate, a phosphonium-based condensing agent such as 1H-benzotriazole-1-yloxytris(dimethylamino)phosphonium hexafluorophosphate, a uronium-based condensing agent such as o-(benzotriazole-1-yl)-N,N,N',N'-tetramethyluronium hexafluorophosphate, and a haulonium-based condensing agent such as 2-chloro-1,3-dimethylimidazolinium hexafluorophosphate.

The condensing agent may be in the form of a salt (for example, hydrochloride).

For example, the condensing agent in the disclosure preferably includes a carbodiimide-based condensing agent.

The condensing agent is preferably a carbodiimide compound represented by the following Formula (Z) or a hydrochloride of a carbodiimide compound containing an amino group among carbodiimide compounds represented by the following Formula (Z).

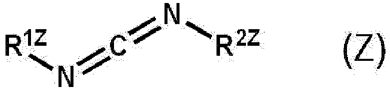

In Formula (Z), each of R^{1Z} and R^{2Z} independently represents a hydrocarbon group having from 1 to 10 carbon atoms which may be substituted with an amino group.

The amino group is a concept including both a substituted amino group and an unsubstituted amino group.

The amino group in the disclosure includes, for example, a monoalkylamino group, a dialkylamino group, or the like.

The "carbodiimide compound containing an amino group among carbodiimide compounds represented by Formula (Z)" is specifically a carbodiimide compound represented by Formula (Z), and in Formula (Z), R^{1Z} and R^{2Z} are each independently a hydrocarbon group having from 1 to 10 carbon atoms which may be substituted with an amino group and at least one of R^{1Z} and R^{2Z} may be a carbodiimide compound which is substituted with an amino group.

The "hydrochloride of a carbodiimide compound containing an amino group among carbodiimide compounds represented by Formula (Z)" means a hydrochloride of the "carbodiimide compound containing an amino group among carbodiimide compounds represented by Formula (Z)".

The number of carbon atoms of the hydrocarbon group in R^{1Z} or R^{2Z} means the total number of carbon atoms contained in R^{1Z} or R^{2Z}.

For example, in a case in which R^{1Z} or R^{2Z} include a substituted amino group, the number of carbon atoms of the hydrocarbon group in R^{1Z} or R^{2Z} also includes the number of carbon atoms of the substituted amino group.

The condensing agent is more preferably a carbodiimide compound represented by the following Formula (Z 1) or a hydrochloride thereof:

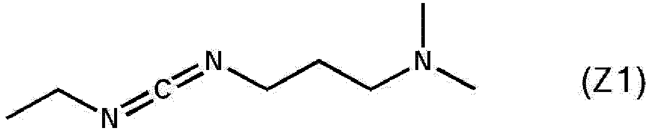

More specific examples of the condensing agent include 1-(3-dimethylaminopropyl)-3-ethylcarbodiimide (also referred to as EDC), 1-(3-dimethylaminopropyl)-3-ethylcarbodiimide hydrochloride (also referred to as EDC·HCl), N,N'-dicyclohexylcarbodiimide, and N,N'-diisopropylcarbodiimide. Among them, 1-(3-dimethylaminopropyl)-3-ethylcarbodiimide and 1-(3-dimethylaminopropyl)-3-ethylcarbodiimide hydrochloride are preferable as the condensing agent.

### (Primary Amine Compound)

The production method of the disclosure uses a primary amine compound.

The primary amine compound in the disclosure is not particularly limited.

The primary amine compound in the disclosure may be a monoamine compound or a diamine compound, but is preferably a diamine compound. The diamine compound may include, for example, a cyclic structure or may not include a cyclic structure, but preferably includes a cyclic structure.

The primary amine compound in the disclosure is more preferably a primary amine compound including two amino groups and a cyclic structure from the viewpoint that water absorbability can be suppressed in the case of curing the synthesized (meth)acrylamide compound (X).

In a case in which the primary amine compound in the disclosure includes a cyclic structure, the cyclic structure is not particularly limited.

For example, the cyclic structure may be an alicyclic structure or an aromatic structure, but an alicyclic structure is preferable.

The number of carbon atoms in the cyclic structure is preferably from 4 to 15 and more preferably from 6 to 13.

The primary amine compound may include one cyclic structure or two or more cyclic structures.

In the primary amine compound, the cyclic structure and two amino groups may be bonded directly or via a divalent linking group.

Examples of the divalent linking group include a methylene group and an ethylene group, and a methylene group is preferable.

The primary amine compound preferably includes a compound represented by any one of the following Formulas (1-1) to (1-7):

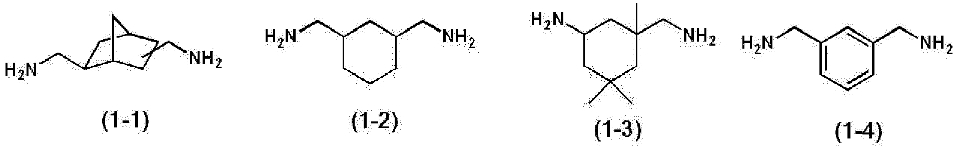

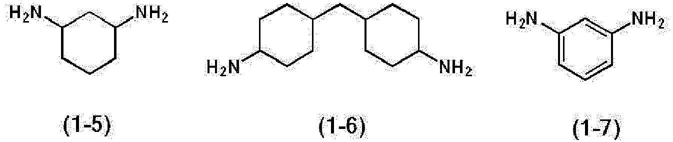

### <(Meth)Acrylamide Compound (X)>

The (meth)acrylamide compound (X) in the disclosure is synthesized by reacting a (meth)acrylic acid with a primary amine compound.

A (meth)acrylamide compound (X) preferably includes a cyclic structure and a (meth)acrylamide group.

When the (meth)acrylamide compound (X) has the structure, a cured product in which an increase in water absorption rate is suppressed can be obtained. As a result, a cured product in which the bending strength is maintained can be obtained.

In the cured product in the disclosure, since the bending strength is favorably maintained, for example, the elastic modulus and breaking strength of the cured product can be favorably maintained.

The (meth)acrylamide compound (X) is preferably a compound represented by the following Formula (1).

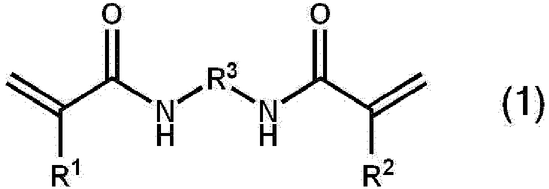

In Formula (1), each of R¹ and R² independently represents a hydrogen atom or a methyl group, and R³ represents a divalent group including two amino groups and a cyclic structure.

In Formula (1), the details of the specific aspects and the like of the primary amine compound including two amino groups and a cyclic structure are the same as the details of the specific aspects and the like of the primary amine compound described above.

R³ in Formula (1) is preferably a group represented by the following Formula (1a).

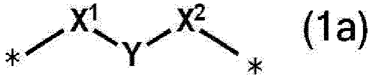

In Formula (1a), each of X¹ and X² independently represents a single bond or a methylene group, Y represents a divalent linking group having from 6 to 13 carbon atoms including an alicyclic structure or an aromatic structure, and each of two *'s represents a bonding position.

In Formula (1a), the divalent linking group in Y preferably has from 6 to 10 carbon atoms and more preferably from 6 to 8 carbon atoms.

The molecular weight of the (meth)acrylamide compound (X) is preferably from 150 to 500, more preferably from 200 to 400, and still more preferably from 200 to 350.

It is more preferable that the (meth)acrylamide compound (X) of the disclosure contains two (meth)acrylamide groups and a divalent alicyclic hydrocarbon group, in which nitrogen atoms in the two (meth)acrylamide groups are both bonded to the divalent alicyclic hydrocarbon group via a methylene group.

The (meth)acrylamide compound (X) can be produced, for example, by reacting a (meth)acrylic compound (X), which is at least one selected from the group consisting of a (meth)acrylic acid and a halide of a (meth)acrylic acid with a primary amine compound (Y1) that includes two amino groups and the divalent alicyclic hydrocarbon group, in which nitrogen atoms in the two amino groups being both bonded to the divalent alicyclic hydrocarbon group via a methylene group.

### <<Monomer Composition>>

The synthesis step in the disclosure may be to synthesize a (meth)acrylamide compound (X) by reacting a (meth)acrylic acid with a primary amine compound in the presence of a condensing agent, thereby obtaining a composition (also referred to as a monomer composition) containing the (meth)acrylamide compound (X).

The monomer composition in the disclosure may contain a component other than the (meth)acrylamide compound, but it is preferable that the monomer composition does not substantially contain any components other than the (meth)acrylamide compound.

Therefore, the timing of commencement of the polymerization can be adjusted. That is, in a case in which the monomer composition does not substantially contain any component other than the (meth)acrylamide compound, curing is not started, and polymerization can be started by adding a polymerization initiator at a desired time.

From the above viewpoint, for example, in the monomer composition in the disclosure, the content of the (meth)acrylamide compound (X) is preferably 90% by mass or more and more preferably 95% by mass or more with respect to the total mass of the monomer composition.

In the monomer composition in the disclosure, the content of the (meth)acrylamide compound (X) may be 100% be mass or less or 99% by mass or less with respect to the total mass of the monomer composition.

### [Purification]

In the production method of the disclosure, the synthesizing a (meth)acrylamide compound (X) (that is, the synthesis step) is to obtain a composition containing the (meth)acrylamide compound (X), and the production method may include purifying the composition containing the (meth)acrylamide compound (X) after the synthesis step.

Since the production method of the disclosure does not require complicated purification such as column purification, the purification can be a simple purification. Examples of the purification include purification by water washing.

Specifically, the purification by water washing may be in the following aspect.

In the production method of the disclosure, it is preferable that the synthesis step is to obtain a composition containing the (meth)acrylamide compound (X), and the production method includes subjecting the composition containing the (meth)acrylamide compound (X) to acid washing and alkali washing (also referred to a water washing step) after the synthesis step.

When the production method of the disclosure includes the water washing step, the (meth)acrylamide compound (X) can be purified without performing complicated purification such as column purification, so that convenience can be improved.

In the water washing step, the acid washing and the alkali washing may be each independently performed once or more, or may be performed twice or more.

The monomer composition of the disclosure is preferably used for a dental material.

Examples of the dental material include a dental restorative material, a denture base resin, a denture base lining material, an impression material, a joint wearing material (such as resin cement or resin-added glass ionomer cement), a dental adhesive (such as an orthodontic adhesive or a cavity coating adhesive), a tooth fissure sealant, a resin block for CAD/CAM, temporary crown, and an artificial tooth material.

Examples of the dental restorative material include a composite resin for a crown, a composite resin for filling a carious cavity, a composite resin for abutment construction, and a composite resin for filling restoration.

### Examples

Hereinafter, the disclosure will be more specifically described with reference to Examples; however, the disclosure is not limited to the following Examples.

Abbreviations of compounds used in Examples of the disclosure are shown below.
EDC·HCl: 1-(3-dimethylaminopropyl)-3-ethylcarbodiimide hydrochloride
DCM: dichloromethane
TEA: triethylamine
MACl: methacrylic acid chloride
AcOEt: ethyl acetate
MeOH: methanol
NBDA: bis(aminomethyl)norbornane
XDA: m-xylylenediamine
DODA: 1,2-bis(2-aminoethoxy)ethane
H6XDA: 1,3-bis(aminomethyl)cyclohexane
IPDA: isophorone diamine
D-400: JEFFAMINE (registered trademark) D-400
D-2000: JEFFAMINE (registered trademark) D-2000
UDMA: 2,2,4-trimethylhexamethylene bis(2-carbamoyloxyethyl) dimethacrylate
3G: triethylene glycol dimethacrylate
CQ: camphorquinone
DMBE: 2-butoxyethyl 4-(dimethylamino) benzoate

Structural formulas of NBDA, H6XDA, IPDA, XDA, D-400, D-2000, and DODA are as follows.

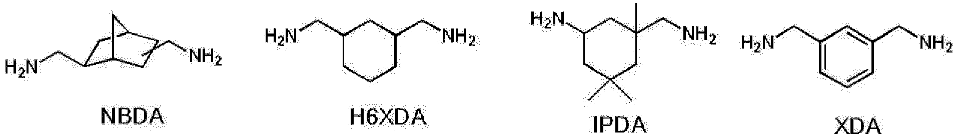

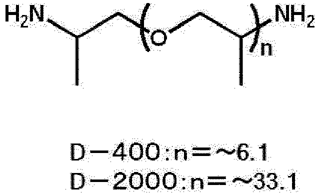

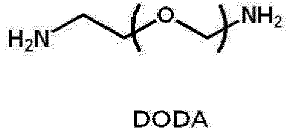

### [Method of Measuring HPLC]

The HPLC chart spectrum of the (meth)acrylamide compound obtained in each Example or Comparative Example was measured using an HPLC apparatus: LC-20AT manufactured by SHIMADZU CORPORATION.

After dissolving the (meth)acrylamide compound obtained in each Example or Comparative Example in CH₃CN, the (meth)acrylamide compound was measured with an eluent of CH₃CN/H₂O = 90/10.

### [Method of Measuring IR Spectrum]

The IR spectrum of the (meth)acrylamide compound obtained in each Production Example was measured using Fourier Transform Infrared Spectroscopy, Spectrum Two/UATR (Universal Attenuated Total Reflectance) manufactured by PerkinElmer Japan Co., Ltd.

The obtained (meth)acrylamide compound was left to stand still at 20°C for 24 hours, and then the infrared absorption spectrum of the (meth)acrylamide compound was measured at 20°C.

### [Example 1: Condensation of NBDA + Methacrylic Acid]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 15.43 parts by mass of NBDA was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6 hours while keeping the reaction temperature at 30°C. The reaction mass was a colorless clear liquid. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The treatment time of the water washing step was 1 hour. The remaining DCM layer was distilled off with an evaporator for 2 hours to obtain methacrylamide (1) as a white solid. The total treatment time was 3 hours. The yield according to this production method was 21.0 g. The yield, the purity, the reaction time, and the treatment time are summarized in Table 1.

The methacrylamide (1) was subjected to IR spectrum measurement, and the decay of the peak intensity of the amino group at from 3200 cm⁻¹ to 3500 cm⁻¹ was confirmed. The structure of the methacrylamide (8) was confirmed to be the following structure.

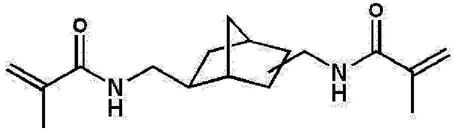

### [Example 2: Condensation of XDA + Methacrylic Acid]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 13.62 parts by mass of XDAwas added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 5 hours while keeping the reaction temperature at 30°C. The reaction mass was a pale yellow clear liquid. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The treatment time of the water washing step was 1.5 hours. The remaining DCM layer was distilled off with an evaporator for 2 hours to obtain methacrylamide (2) as a white solid. The total treatment time was 3.5 hours. The yield according to this production method was 21.1 g. The yield, the purity, the reaction time, and the treatment time are summarized in Table 1.

The methacrylamide (2) was subjected to IR spectrum measurement, and the decay of the peak intensity of the amino group at from 3200 cm⁻¹ to 3500 cm⁻¹ was confirmed. The structure of the methacrylamide (8) was confirmed to be the following structure.

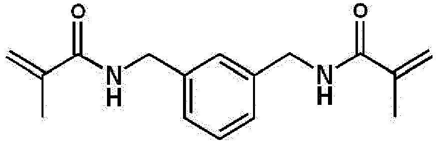

### [Example 3: Condensation of DODA + Methacrylic Acid]

Into a thoroughly dried 300 mL four-necked flask equipped with a stirring blade and a thermometer, 17.22 parts by mass of methacrylic acid and 100 parts by mass of DCM were charged and dissolved. Thereafter, the solution was cooled to 10°C, and 38.34 parts by mass of EDC HCL was charged in a divided manner so that the internal temperature did not exceed 10°C. The mixture was dissolved to form a homogeneous solution, and then reacted at 10°C for 0.5 hours. To the solution, 14.82 parts by mass of DODA was added dropwise over 0.5 hours. The dropwise addition was accompanied by an increase in internal temperature due to the reaction heat, and thus the rate of the dropwise addition was controlled so that the temperature was 30°C or lower. After the whole amount had been added dropwise, the reaction was performed for 6 hours while keeping the reaction temperature at 30°C. The reaction mass was a colorless clear liquid. During this process, the progress of the reaction was tracked by HPLC analysis to determine the end point of the reaction. To the flask, 50 parts by mass of a 5% citric acid aqueous solution was added, the mixture was stirred for 10 minutes, and then the aqueous layer was discharged. The same operation was performed for distilled water, 5% sodium carbonate water, and distilled water in this order. The treatment time of the water washing step was 1 hour. The remaining DCM layer was distilled off with an evaporator for 2 hours to obtain methacrylamide (1) as a white solid. The remaining DCM layer was distilled off with an evaporator for 2 hours to obtain methacrylamide (3) as a pale yellow liquid. The total treatment time was 3 hours. The yield according to this production method was 17.8 g. The yield, the purity, the reaction time, and the treatment time are summarized in Table 1.

The methacrylamide (3) was subjected to IR spectrum measurement, and the decay of the peak intensity of the amino group at from 3200 cm⁻¹ to 3500 cm⁻¹ was confirmed. The structure of the methacrylamide (8) was confirmed to be the following structure.

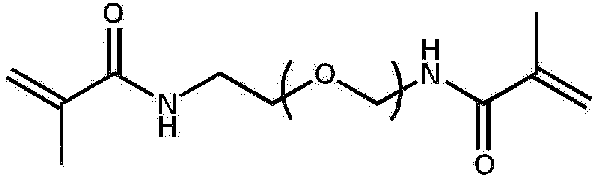

### [Comparative Example 1: Reaction of NBDA + Methacrylic Acid Chloride]

Into a thoroughly dried 300 mL four-necked flask equipped with a stirring blade and a thermometer, 15.43 parts by mass of NBDA, 30.36 parts by mass of TEA, and 100 parts by mass of DCM were charged and dissolved. Thereafter, the solution was cooled to -10°C. To the solution, 31.35 parts by mass of MACl was added dropwise over 0.5 hours. The dropwise addition was accompanied by an increase in internal temperature due to the reaction heat, and thus the rate of the dropwise addition was controlled so that the temperature was 10°C or lower. After the whole amount had been added dropwise, the reaction was performed for 5 hours while keeping the reaction temperature at 10°C. The reaction mass was a red-brown slurry. During this process, the progress of the reaction was tracked by HPLC analysis to determine the end point of the reaction. After the reaction, triethylamine hydrochloride was separated by filtration. The treatment time of the filtration step was 0.5 hours. The filtrate was distilled off with an evaporator for 2 hours to obtain a concentrated residue. The obtained concentrated residue was purified by using silica gel column chromatography (developing solvent: AcOEt/MeOH = 3/1). The time required for column purification was 6 hours. After column purification, the solvent was distilled off with an evaporator for 2 hours to obtain methacrylamide (1) as a white solid. The total treatment time was 10.5 hours. The yield according to this production method was 5.7 g. The yield, the purity, the reaction time, and the treatment time are summarized in Table 1.

### [Comparative Example 2: Reaction of XDA + Methacrylic Acid Chloride]

Into a thoroughly dried 300 mL four-necked flask equipped with a stirring blade and a thermometer, 13.62 parts by mass of XDA, 30.36 parts by mass of TEA, and 100 parts by mass of DCM were charged and dissolved. Thereafter, the solution was cooled to -10°C. To the solution, 31.35 parts by mass of MACl was added dropwise over 0.5 hours. The dropwise addition was accompanied by an increase in internal temperature due to the reaction heat, and thus the rate of the dropwise addition was controlled so that the temperature was 10°C or lower. After the whole amount had been added dropwise, the reaction was performed for 4.5 hours while keeping the reaction temperature at 10°C. The reaction mass was a dark-brown slurry. During this process, the progress of the reaction was tracked by HPLC analysis to determine the end point of the reaction. After the reaction, triethylamine hydrochloride was separated by filtration. The treatment time of the filtration step was 0.5 hours. The filtrate was distilled off with an evaporator for 2 hours to obtain a concentrated residue. The obtained concentrated residue was purified by using silica gel column chromatography (developing solvent: AcOEt/MeOH = 3/1). The time required for column purification was 7.5 hours. After column purification, the solvent was distilled off with an evaporator for 2 hours to obtain methacrylamide (2) as a white solid. The total treatment time was 12 hours. The yield according to this production method was 4.0 g. The yield, the purity, the reaction time, and the treatment time are summarized in Table 1.

### [Comparative Example 3: Reaction of DODA+ Methacrylic Acid Chloride]

Into a thoroughly dried 300 mL four-necked flask equipped with a stirring blade and a thermometer, 14.82 parts by mass of DODA, 30.36 parts by mass of TEA, and 100 parts by mass of DCM were charged and dissolved. Thereafter, the solution was cooled to -10°C. To the solution, 31.35 parts by mass of MACl was added dropwise over 0.5 hours. The dropwise addition was accompanied by an increase in internal temperature due to the reaction heat, and thus the rate of the dropwise addition was controlled so that the temperature was 10°C or lower. After the whole amount had been added dropwise, the reaction was performed for 6 hours while keeping the reaction temperature at 10°C. The reaction mass was a red-brown slurry. During this process, the progress of the reaction was tracked by HPLC analysis to determine the end point of the reaction. After the reaction, triethylamine hydrochloride was separated by filtration. The treatment time of the filtration step was 0.5 hours. The filtrate was distilled off with an evaporator for 2 hours to obtain a concentrated residue. The obtained concentrated residue was purified by using silica gel column chromatography (developing solvent: AcOEt/MeOH = 3/1). The time required for column purification was 5 hours. After column purification, the solvent was distilled off with an evaporator for 2 hours to obtain methacrylamide (3) as a pale yellow liquid. The total treatment time was 9.5 hours. The yield according to this production method was 4.0 g. The yield, the purity, the reaction time, and the treatment time are summarized in Table 1.

**[Table 1]**

| | | Example 1 | Comparative Example 1 | Example 2 | Comparative Example 2 | Example 3 | Comparative Example 3 |
|---|---|---|---|---|---|---|---|
| Raw material amine | | NBDA | | XDA | | DODA | |
| Synthesis method | | Condensation | Acid chloride | Condensation | Acid chloride | Condensation | Acid chloride |
| Reaction | Reaction time [hr] | 6 | 5 | 5 | 4.5 | 6 | 6 |
| | Reaction mass hue | Colorless | Red-brown | Pale yellow | Dark-brown | Colorless | Red-brown |
| | Reaction mass property | Clear liquid | Slurry | Clear liquid | Slurry | Clear liquid | Slurry |
| Purification | Filtration [hr] | - | 0.5 | - | 0.5 | - | 0.5 |
| | Concentrating [hr] | - | 2 | - | 2 | - | 2 |
| | Column [hr] | - | 6 | - | 7.5 | - | 5 |
| | Water washing [hr] | 1 | - | 1.5 | - | 1 | - |
| | Concentrating [hr] | 2 | 2 | 2 | 2 | 2 | 2 |
| | Total time [hr] of purification | 3 | 10.5 | 3.5 | 12 | 3 | 9.5 |
| Yield [%] | | 72.3 | 19.7 | 77.5 | 14.7 | 62.7 | 25.6 |
| Purity [%] | | 97.3 | 95.6 | 96.8 | 96.1 | 97.4 | 95.3 |

As shown in Table 1, in Examples using the method for producing a (meth)acrylamide compound, including synthesizing a (meth)acrylamide compound (X) by reacting a (meth)acrylic acid with a primary amine compound in the presence of a condensing agent, the (meth)acrylamide compound (X) can be obtained without performing complicated purification such as column purification. Therefore, the (meth)acrylamide compound could be produced by a simple method.

On the other hand, in Comparative Examples 1 to 3 in which a (meth)acrylamide compound was synthesized by an acid chloride method instead of a condensation reaction, the (meth)acrylamide compound could be obtained, but column purification was performed, and the (meth)acrylamide compound could not be produced by a simple method.

Example 1 was superior in purity to Comparative Example 1, Example 2 was superior to Comparative Example 2, and Example 3 was superior to Comparative Example 3.

Example 1 was remarkably superior in yield to Comparative Example 1, Example 2 was superior to Comparative Example 2, and Example 3 was superior to Comparative Example 3.

Coloring was suppressed more in Example 1, Example 2, and Example 3 as respectively compared with Comparative Example 1, Comparative Example 2, and Comparative Example 3.

The entire contents of the disclosures by Japanese Patent Application No. 2021-152361, filed September 17, 2021 are incorporated herein by reference.

All the literature, patent application, and technical standards cited herein are also herein incorporated to the same extent as provided for specifically and severally with respect to an individual literature, patent application, and technical standard to the effect that the same should be so incorporated by reference.

## Claims

1. A method for producing a (meth)acrylamide compound, comprising: synthesizing a (meth)acrylamide compound (X) by reacting a (meth)acrylic acid with a primary amine compound in the presence of a condensing agent.

2. The method for producing a (meth)acrylamide compound according to claim 1, wherein the condensing agent is a carbodiimide compound represented by the following Formula (Z) or a hydrochloride of a carbodiimide compound containing an amino group among carbodiimide compounds represented by the following Formula (Z): 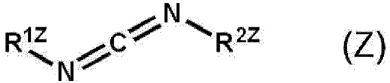 wherein, in Formula (Z), each of R^{1Z} and R^{2Z} independently represents a hydrocarbon group having from 1 to 10 carbon atoms that may be substituted with an amino group.

3. The method for producing a (meth)acrylamide compound according to claim 1, wherein the condensing agent is a carbodiimide compound represented by the following Formula (Z1) or a hydrochloride thereof:

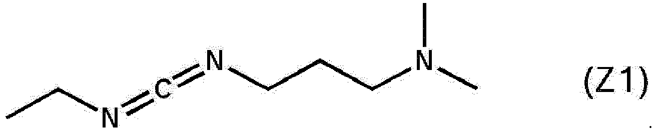

4. The method for producing a (meth)acrylamide compound according to claim 1, wherein the primary amine compound is a diamine compound.

5. The method for producing a (meth)acrylamide compound according to claim 1, wherein the synthesizing a (meth)acrylamide compound (X) obtains a composition containing the (meth)acrylamide compound (X), and
the method further comprises, after the synthesizing a (meth)acrylamide compound (X), subjecting the composition containing the (meth)acrylamide compound (X) to acid washing and alkali washing.
